# EUROPEAN PATENT SPECIFICATION

(11) **EP 0 652 744 B1**
(45) Date of publication and mention of the grant of the patent: **20.03.1996**
(21) Application number: 93917634.3
(22) Date of filing: 23.07.1993
(51) Int. Cl.: A61K 9/127, A61K 9/107, A61K 9/06

(54) **PHARMACEUTICAL COMPOSITIONS FOR TRANSMUCOSAL DELIVERY OF PEPTIDES**
ARZNEIMITTEL ZUR TRANSMUCOSALEN VERABREICHUNG VON PEPTIDEN
COMPOSITIONS PHARMACEUTIQUES UTILES DANS L'APPORT TRANSMUQUEUX DE PEPTIDES

(30) Priority: 28.07.1992 IT MI921831
(43) Date of publication of application: 17.05.1995
(73) Proprietor: POLI INDUSTRIA CHIMICA S.p.A., I-20141 Milano (IT)
(72) Inventor: POLI, Stefano, I-20089 Quinto de' Stampi-Rozzano (IT); MAILLAND, Federico, I-20089 Quinto de' Stampi-Rozzano (IT); MORO, Luigi, I-20089 Quinto de' Stampi-Rozzano (IT)
(74) Representative: Bianchetti, Giuseppe
(86) International application number: EP9301965
(87) International publication number: WO9403157

(56) References cited:
- EP-A- 0 177 223
- EP-A- 0 386 960
- WO-A-90/00048
- FR-A- 2 660 192
- US-A- 4 944 948

## Description

The present invention refers to pharmaceutical compositions in the form of microemulsions or liposomic dispersions for the transmucousal administration of proteins or peptidic substances pharmacologically active; they differ from the known liposomic or microemulsified compositions in that they contain in addition a thermosetting agent able to enhance the residence time on the administration site and, consequently, to promote the absorption of the delivered drug.

The administration of proteinic substances has been, from few years ago, limited to the parenteral route as it was the only one which granted a good absorption to molecules having a complex structure and not able to tolerate environments with a high acidity and rich of proteolytic enzymes such as that ones can be found in the digestive apparatus. The need to apply by means of an invasive way, the difficulty in obtaining, on a large scale, many proteins from natural sources and the very high activity of such substances, with the consequent risks of overdosage, have determined the limited diffusion of such substances in the clinical practice.

Recent attempts to orally administer the peptide substances with drug delivery systems such as microparticles and liposomes, did not obtain relevant results in absorption or reproduciblity of the same: the transit time variability in the digestive apparatus, associated to the large presence of proteolytic enzymes, has made the researcher to consider the oral administration as a problem.

More recently, always in the attempt to avoid the more invasive parenteral administration, formulations suitable to the nasal administration of proteinic substances have been proposed.

The tipical disadvantage of this way of administration consists in: the relatively reduced area available for the absorption, the high clearance (which reduce the time of contact) and the particular characteristic of the muconasal epithelium which covers the upper respiratory organs, that is the presence of cilia in association with mucus producing glands.

In addition, the "absorption promoters" frequently used in formulations for nasal administration produce a relevant damage to the mucociliary clearance of the deposit zone of the formulation and give problems to the repeated or chronic treatment cycles.

With the same aims,it has also been proposed the rectal administration of protein substances: this kind of administration is nevertheless confined in the mediterranean area and even considered unproposable to the nordamerican and nordeuropean populations. In addition to the ethnical inconvenient, is to be considered that the rectal ampoul has a limited surface and a basic pH.

More recently it has been found that the intravaginal way, till now considered for local treatment only, can be used to allow the systemic absorption of protein substances. The vaginal mucous is in fact able to allow the diffusion of substances pharmacologically active from the application surface to the dermic stratum where is present a rich vascular area able to absorb and drain in the systemic circle, besides the deep dermic stratum, considerable quantity of the substance applied.

An important characteristic wich allow high absorption values in the vaginal area, as well as for nasal cavity and mucous in general, is the bioadhesion of the formulation.

The formulations commonly used in the medical practice take advantage of the vehicle charachteristics: its viscosity and composition play an important role on the time of persistence of the drug effective amount in the absorption area, as well as the achievement of an opportune absorption area and the extension of the same.

Generally, the lower is the mobility of the vehicle, the higher is its viscosity then, the more permanence time increases, consequently, higher is the possibility that active substance is quantitatively absorbed.

Nevertheless, we must not neglect the fact that, usually, a reduced viscosity helps to spread the dispersion of the dosage applied by means of suitable mechanical devices, such as the nasal minipump or foam generators, allowing a very fine distribution of the product. This is very important to increase the contact surface and promote drug absorption.

The objects of present invention are pharmaceutical compositions in the form of microemulsions or liposomic dispersions characterized by the fact that contain a thermosetting agent able to allow the product viscosity increase with temperature, thus allowing a longer mucousal residence time and enhanced drug absorption profile.

Due to the thermosetting properties of the vehicles it is possible to make pharmaceutical compositions which have a reduced viscosity at room temperature, helping the distribution on a larger surface, with a product finely divided. When the composition reach the mucous a structural change take place as a function of the body temperature, the viscosity of the product increase thus determining a large persistence of the system on the absorption zone.

The use of formulations that are liquid at room temperature but which increase their viscosity with temperature giving semi-solid products when warmed to the body value is already known.

There are, in fact, some patents that describe the use of a particular polymer (Pluronic) to reach that goal.

As example, the U.S. Pat. No. 4,478,822 describes a vehicle useful to deliver a medicament to a body orifice, with a drug delivery system consisting of a clear liquid which forms semi-solid gel at human body temperature.

The desired sol-gel transition temperature of the solution can be modified by changes in polymer concentration or in chemical characteristics of the solution.

EP-A-0177223 discloses a delivery system comprising a dispersion of liposomes viscosized with hydrogels, e.g. methylcellulose.

We have surprisingly found that the same goal can be obtained also in systems more and more complex, like liposomes of microemulsions.

Normally, this type of formulations require an hard work to balance the composition. In fact, microemulsions are a very complex system with the coexistence of at least three different phases: a disperse phase, an interface layer of surfactants and/or cosurfactants surrounding the disperse phase, a continuous phase that contains the previous ones. The addition of relevant amounts of copolymer in order to obtain the sol-gel system transition, normally change dramatically the precise ratio between the 4 main components of the microemulsion system, that are water, surfactants, cosurfactants and oil. Only a formulation work devoted to find a new balance point allows to obtain thermosetting microemulsions. The same problem, with due proportions and limitations, is arising also to liposomic dispersion.

In a typical realization, the invention uses as thermosetting vehicle, a polyoxyethylene-polyoxypropylene copolymer, preferably the one known with the trade name of Pluronic F 127™ or Lutrol F 127™. These characteristics, which are favourable even when present in conventional solutions, are particularly important and effective when join complex and modern vehicles such as the liposomic or microemulsion systems.

The liposomic systems, well-known for their potential of vectorization, consist in spherules, with very small dimensions, composed of concentric layers of phospholipidic material which are alternated to discrete and isolated aqueous spaces.

The high affinity with mucouses, typical of liposomic systems, is dramatically increased by the introduction of termosetting polymers, generating a product with bioadhesive characteristics which, by increasing the potential of vectorisation, leads to an improved activity.

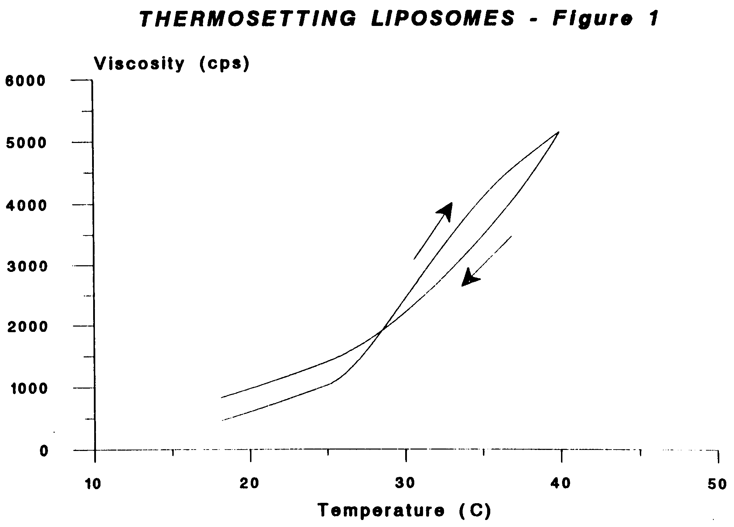

For the liposomic products, that contain the polyoxyethylene-polyoxypropylene copolymer, the viscosity changement induced by the temperature is reversible (fig. 1).

What previously said can be extended to the microemulsionated systems too: the well proportioned combination of its constituent, essential from a structural point of view, with a polyoxyethylene-polyoxypropylene copolymer produces an "apparent solution" able to increase the viscosity when in contact with mucouses. The human body application of this system, following the partial solvent evaporation, generates a barrier which, owing to the thermosetting gelation, promotes the bioadhesion of the system.

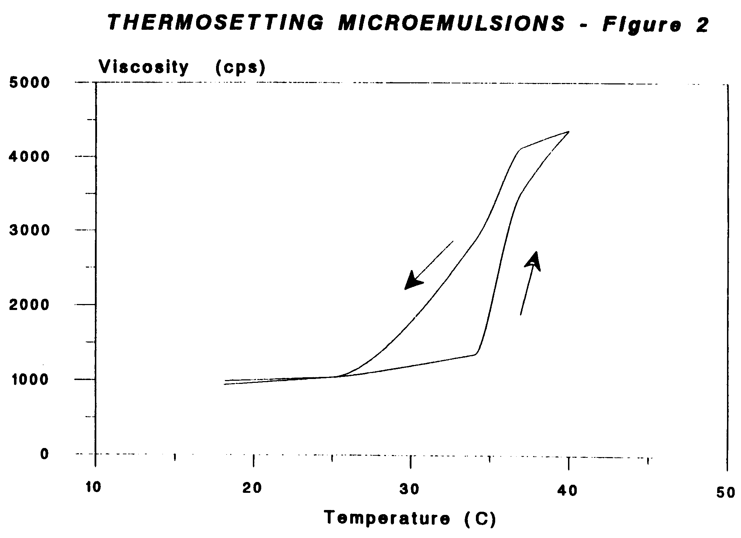

Even in this case the transition temperature from the sol condition to the gel is reversible (fig. 2).

Object of the present invention are pharmaceutical compositions useful for the transmucousal administration, in particular vaginal or nasal, of proteinic or peptidic substances pharmacologically active. Examples of similar substances include calcitonin, insulin, desmopressine, interleukin, interferon, GMCSF (granulocite monocite colony stimulating factor), ciclosporin, posatirelin, protirelin, timopentin, pidotimod, mono or polyclonales antigenes, antigenic proteins of bacterial or viral origin, parathormon, gonadorelin, coagulation factors, epidermic growth factors, "insulin like" growth factor, endorphin and their derivatives or fragments, tioxoprolylcysteine, tioxoprolylthiazolidincarboxylic acid, irudine and their derivatives.

The pharmaceutical compositions, in accordance to the invention, can be in any form suitable for vaginal or nasal administration, such as soft gelatine vaginal capsules, vaginal suppository composed of natural or semi-synthetic glycerides, creams, gels, emulsions, suspensions, solutions, foams.

The liposomic systems can contain:
- modulators of transition temperature of phospholipids, such as cholesterol and its derivatives;
- antioxidant agents such as tochopherols, and their esters, BHA, BHT, carotenes;
- stabilizer agents;
- preservatives;
- an alcoholic solvent phase;
- eventual auxiliary substances, such as pH correctors, moisturizers, perfumes, essences.

The microemulsion systems, in addition to the components already mentioned and to the polyoxyethylene-polyoxypropylene copolymer, could contain excipients known by the experts with the names of antioxidant, stabilizer, preservatives, buffers, and so on.

Obviously, the administration of liposomic and microemulsionated systems will be made easier by the use of suitable spray dispensers or applicators in form of cannula, syringe or similar devices.

According to a preferred embodiment of the invention, the compositions will contain stabilizer or absorption promoter, polygalacturonic acid, polyglucuronic acid, hyaluronic acid, hyaluronamine, hyaluronamide or their salts and pharmaceutical acceptable derivatives.

In vivo experimental studies employing products relevant to the invention, have shown that intravaginal or nasal route allow a systemic bioavailability comparable with that ones achievable by a parenteral administration, without presenting the limits of this route. As a consequence the dosage potency of proteins or peptidic substances farmacologically active by vaginal or nasal administration will be substantially similar to those already used for the well-known administration routes. It is possible that, using pharmaceutical forms not exactly metered, such as solutions, creams or gel, according to the invention, it is possible to obtain a personalization of the dosage: considering the often high pharmacological activity of the protein substances, this gives important advantages related to the reduction of the risks of overdose.

According to a preferred embodiment, the invention gives pharmaceutical compositions suitable to the vaginal or nasal administration containing as active ingredient a calcitonin of any source.

The high bioavailability level achievable by the vaginal administration of a calcitonin products relevant to the invention, with a proper bioadhesion, is shown by a pharmacological experiment. The decrease of calcemic level in rabbit serum after vaginal application of: a) a simple solution, b) a thermosetting gel and c) a thermosetting gel furtherly thickned with hyaluronic acid, has been measured. As a reference the decrease of calcemic serum level obtained by administration of an equal dose of the same drug by i.m. route (see tab. 1) was chosen.

**Table 1**

| **ADMINISTRATION** | | **CALCEMIC LEVEL VS BASAL VALUE** | |
|---|---|---|---|
| TYPE | ROUTE | AREA (cm²) | % VS I.M. |
| Solution 100 I.U. | i.m. | 444 | 100 |
| Solution 100 I.U. | vaginal | 264 | 60 |
| Gel(Pluronic F127) 100 I.U. | vaginal | 439 | 99 |
| Gel(High m.w. Hyalur.Acid)100 I.U. | vaginal | 425 | 95 |

The demonstration of a systemic absorption of proteic substances, when administrated by vaginal or nasal route employing liposomic or microemulsions bioadhesive preparations, make possible a vaccine-therapy uninvasive or other forms of immunitary protection using antigen substances.

It is well-known that the oral administration of drugs meets the compliance of patients; but it is well-known too that a simple pharmaceutical oral dosage form cannot be employed with peptides or proteins. The object of the present invention is a pathway to bypass the limits of the oral route keeping a good compliance of the patients.

Neverthless the thermosetting liposomes or microemulsions not only are a drug delivery system particularly usefull for peptides and proteins, but can be advantageously employed also for low molecular weight drug, like nicotine, FANS and so on.

The invention will have a more detailed description by the following examples.

### Example 1

Lecithin (4 g), and cholesterol (0.75 g) were dissolved in ethyl alcohol. Tocopherol acetate (0.02 g) was added to the solution. In an other container, sodium methylparaben (0.15 g), edetate disodium (0.1 g) and salmon calcitonin (7 mg) were dissolved in purified water (80 mL). The aqueous solution was added to the first one under stirring. The alcohol was evaporated by heating to form a liposomic dispersion . Thereto were added Lutrol F127 (13 g) and purified water (q.s.to reach 100 mL). The liposomic dispersion was subdivided in glass vials that following were closed with a minipump. A pre-arranged unit dose administration was so allowed.

### Example 2

Soybean lecithin (30 g), tocopherol acetate (500 mg) and cholesterol (2 g) were dissolved by heating in isopropyl alcohol. The solution was keeped at 50°C until 50 mM citrate buffer (pH 4.5, 1000 mL) containing calcitonin (50 I.U./mL), beforehand heated at the same temperature, was added, to give a hydro-alcoholic phospholipid dispersion. The mixture was vigorously shaken under reduced pressure causing the evaporation of isopropyl alcohol and giving a liposomic dispersion of calcitonin. Hydroxyethylcellulose (10 g) was added and a gel, having a suitable viscosity for the vaginal application, was obtained.

### Example 3

Hyaluronic acid sodium salt (10 g) and Posatirelin (3.33 g) were dissolved in 50 mM citrate buffer (pH 4.5, 1000 mL). A suitable amount of Pluronic F127 was added, so to obtain an increased viscosity to body temperature. The gel formed shows a good clearless and can be applied into the vagina syringe dispenser.

### Example 4

Lecithin (6 g) was dissolved in a mixture of isopropyl myristate and ethyl alcohol (12.5 mL). After complete dissolution tocopherol acetate (0.02 g) was added. Thereto sodium cholate (4 g) was suspended. Into an other container sodium methylparaben (0.15 g) and calcitonin (7 mg) were dissolved in purified water (60 mL). Aqueous solution of calcitonin was added to lipophylic phase, maintained vigorously shaked. To the formed microemulsion, Pluronic F127 (15 g) was added and dissolved. A necessary amount of purified water to make the entire amount 100 mL was added. The microemulsion was optically clearless and shown a transition temperature sol-gel of about 30-40°C.

### Example 5

Edetate disodium (0.15 g), sodium methylparaben (0.225 g) and salmon calcitonin (12 mg) were dissolved in purified water (100 mL). A solution, obtained dissolving lecithin (5.6 g), cholesterol (1.12 g) and tocopherol in slightest amount needed of ethyl alcohol, was added. Thereto polyoxyethylene-polyoxypropylene copolymer (Pluronic F127, 19.5 g) was dissolved, and a necessary amount of purified water to make the entire amount 150 mL was added. The liposomic dispersion was subdivided into aerosol pressurizated container giving a thermosetting liposomic foam.

### Example 6

Lecithin (30 g) and nicotine (3.8 g) were dissolved in a mixture of isopropylmiristate and ethyl alcohol, keeping at 50°C until dissolution. After complete dissolution tocopherol acetate (0.6 g) was added and sodium cholate (15 g) was suspended. Into an other container sodium methylparaben (0.5 g) and sodium edetate (0.5 g) were dissolved in pre-heated (60°C) purified water (300 mL). Aqueous solution was added to lipophilic phase, maintained vigorously shaked. The formed microemulsion was cooled to 5-10°C then Pluronic F127 (75 g) was added and dissolved. A necessary amount of purified water to make the entire amount 500 mL was added. The microemulsion, that shown a transition temperature sol-gel of about 30-40°C, can be applied on the skin or on the nasal mucousal with a suitable device.

## Claims

1. Pharmaceutical compositions suitable to drug protein or peptide administration on body mucosal surface characterized in that they are composed by:
a) a multiphasic pharmaceutical administration system containing the active drug substance;
b) a thermosetting polymer or copolymer able to enhance the viscosity of the system after the exposition to the body temperature;

2. Pharmaceutical compositions as described in the claim 1, characterized in that the multiphasic pharmaceutical administration system is represented by a liposomic suspension or dispersion.

3. Pharmaceutical composition as described in the claim 1, characterized in that the multiphasic pharmaceutical administration system is represented by a microemulsion or other pluriphase system containing at least a dispersed oily phase and a continuous aqueous phase or a dispersed aqueous phase and a continuous oily phase.

4. Pharmaceutical compositions as defined in the preceeding claims, characterized in that these are suitable to promote protein or peptide drugs absorption through the skin or mucousal membranes.

5. Pharmaceutical compositions as defined in the preceeding claims, characterized in that these are suitable to promote protein or peptide drugs absorption through the vaginal mucousal tissue.

6. Pharmaceutical compositions as defined in the preceeding claims, characterized in that these are suitable to promote protein or peptide drugs absorption through the nasal mucousal tissue.

7. Pharmaceutical compositions as defined in the preceeding claims, characterized in that these are suitable to promote protein or peptide drugs absorption through the rectal mucousal tissue.

8. Pharmaceutical compositions as specified in the preceeding claims, characterized by the presence of polyoxyethylene-polyoxypropylene copolymers as thermosetting agents (able to increase the product viscosity by mean of the sol-gel phase transition induced by the exposition to the temperature of the body administration site).

9. Pharmaceutical compositions according to claim 8, where the thermosetting agent is represented by a copolymer known with the trade name of PLURONIC F127™, in a final concentration range between 10% and 30% w/w, preferentially between 13% and 20% w/w.

10. Pharmaceutical composition according to the preceeding claims, containing a protein or peptide drug selected from the group consisting of calcitonin, insulin, desmopressine, interleukin, interferon, GMCSF (granulocite monocite colony stimulating factor), ciclosporin, posatirelin, protirelin, timopentin, mono or polyclonal antigens, bacterial or viral antigenic protein, parathormon, gonadorelin, coagulation factors, epidermic growth factors, "insuline like" growth factor, endorphin and derivatives or fragment, thioxoprolylcysteine, tioxoprolilthiazolidincarboxylic acid, nicotine, irudine and/or their derivatives.

11. Pharmaceutical compositions according to the preceeding claims, where the protein or peptide drug is represented by a calcitonin.

12. Pharmaceutical composition according to the preceeding claims, where the drug is represented by an antigen or an antibody suitable to be used for immunotherapy.

13. Pharmaceutical compositions according to the preceeding claims, containing as absorption enhancers or stabilizers one or more of the biopolymers polygalacturonic acid, polyglycuronic acid, hyaluronic acid, hyaluronamide or their salts or derivatives.

14. Pharmaceutical compositions according to the preceeding claims able to be administered as dermal or vaginal foam.

## Patentansprüche

1. Pharmazeutische Zusammensetzungen, die geeignet sind für die Verabreichung Von Arzneimittel-Protein oder -Peptid auf die Oberfläche einer Körperschleimhaut, dadurch gekennzeichnet daß sie bestehen aus
a) einem mehrphasigen pharmazeutischen Verabreichungssystem, welches die aktive Arzneimittelsubstanz enthält; und
b) einem wärmehärtbaren Polymer oder Copolymer, das die Viskosität des Systems nach der Einwirkung der Körpertemperatur erhöhen kann.

2. Pharmazeutische Zusammensetzungen nach Anspruch 1, dadurch gekennzeichnet daß das mehrphasige pharmazeutische Verabreichungssystem durch eine Liposomen-Suspension oder -Dispersion dargestellt wird.

3. Pharmazeutische Zusammensetzungen nach Anspruch 1, dadurch gekennzeichnet, daß das mehrphasige pharmazeutische Verabreichungssystem dargestellt wird durch eine Mikroemulsion oder ein anderes mehrphasiges System, das mindestens eine dispergierte Ölphase und eine kontinuierliche wäßrige Phase oder eine dispergierte wäßrige Phase und eine kontinuierliche Ölphase enthält.

4. Pharmazeutische Zusammensetzungen nach den vorhergehenden Ansprüchen, dadurch gekennzeichnet, daß diese geeignet sind, die Absorption von Protein- oder Peptid-Arzneimitteln durch die Haut oder Schleimhautmembranen zu fördern.

5. Pharmazeutische Zusammensetzungen nach den vorhergehenden Ansprüchen, dadurch gekennzeichnet daß diese geeignet sind, die Absorption von Protein- oder Peptid-Arzneimitteln durch das Vaginalschleimhautgewebe zu fördern.

6. Pharmazeutische Zusammensetzungen nach den vorhergehenden Ansprüchen, dadurch gekennzeichnet, daß diese geeignet sind, die Absorption von Protein- oder Peptid-Arzneimitteln durch das Nasenschleimhaut-Gewebe zu fördern.

7. Pharmazeutische Zusammensetzungen nach den vorhergehenden Ansprüchen, dadurch gekennzeichnet daß diese geeignet sind, die Absorption von Protein- oder Peptid-Arzneimitteln durch das Rektalscheimhaut-Gewebe zu fördern.

8. Pharmazeutische Zusammensetzungen nach den vorhergehenden Ansprüchen, dadurch gekennzeichnet daß sie Polyoxyethylen/Polyoxypropylen-Copolymere als wärmehärtbare Agentien enthalten (die in der Lage sind, die Produkt-Viskosität mittels des Sol-Gel-Phasenüberganges, der durch die Einwirkung der Temperatur des Körpers an der Stelle der Verabreichung induziert wird, zu erhöhen).

9. Pharmazeutische Zusammensetzungen nach Anspruch 8, in denen das wärmehärtbare Agens dargestellt wird durch ein unter dem Handelsnamen PLURONIC F127™ bekanntes Copolymer in einer Endkonzentration in dem Bereich zwischen 10 und 30 Gew./Gew.-%, vorzugsweise zwischen 13 und 20 Gew./Gew.%.

10. Pharmazeutische Zusammensetzungen nach den vorhergehenden Ansprüchen, die ein Protein- oder Peptid-Arzneimittel enthalten, ausgewählt aus der Gruppe, die besteht aus Calcitonin, Insulin, Desmopressin, Interleukin, Interferon, GMCSF (dem Granulozyten-Monozyten-Kolonie-Stimulierungsfaktor), Cyclosporin, Posatirelin, Protirelin, Tirnopentin, mono- oder polyklonalen Antigenen, bakteriellem oder viralem Antigen-Protein, Parathormon, Gonadorelin, Koagulationsfaktoren, Epidermiswachstumsfaktoren, dem "insulinartigen" Wachstumsfaktor, Endorphin und Derivaten oder Fragmenten davon, Thioxoprolylcystein, Thioxoprolylthiazolidincarbonsäure, Nicotin, Irudin und/oder ihren Derivaten.

11. Pharmazeutische Zusammensetzungen nach den vorhergehenden Ansprüchen, in denen das Protein- oder Peptid-Arzneimittel dargestellt wird durch ein Calcitonin.

12. Pharmazeutische Zusammensetzungen nach den vorhergehenden Ansprüchen, in denen das Arzneimittel dargestellt wird durch ein Antigen oder einen Antikörper, das (der) geeignet ist für die Verwendung in, der Immuntherapie.

13. Pharmazeutische Zusammensetzungen nach den vorhergehenden Ansprüchen, die als Adsorptions-Verbesserungsrnittel oder -Stabilisatoren eines oder mehrere der Biopolymeren Polygalacturonsäure, Polyglycuronsäure, Hyaluronsäure, Hyaluronamid oder ihrer Salze oder Derivate enthalten.

14. Pharmazeutische Zusammensetzungen nach den vorhergehenden Ansprüchen, die als Dermal- oder Vaginalschaum verabreicht werden können.

## Revendications

1. Compositions pharmaceutiques convenables pour l'administration de peptide ou de protéine médicamentaux sur la surface d'une muqueuse corporelle, caractérisées en ce qu'elles sont composées par :
(a) un système d'administration pharmaceutique multiphase contenant la substance médicamenteuse active;
(b) un polymère ou un copolymère thermodurcissable capable d'améliorer la viscosité du système après l'exposition à la température du corps.

2. Compositions pharmaceutiques suivant la revendication 1, caractérisées en ce que le système d'administration pharmaceutique multiphase est représenté par une dispersion ou une suspension liposomique.

3. Compositions pharmaceutiques suivant la revendication 1, caractérisées en ce que le système d'administration pharmaceutique multiphase est représenté par une microémulsion ou un autre système pluriphase contenant au moins une phase huileuse dispersée et une phase aqueuse continue, ou une phase aqueuse dispersée et une phase huileuse continue.

4. Compositions pharmaceutiques suivant les revendications précédentes, caractérisées en ce qu'elles conviennent pour favoriser l'absorption de médicaments à base de protéine ou de peptide à travers la peau ou les membranes muqueuses.

5. Compositions pharmaceutiques suivant les revendications précédentes, caractérisées en ce qu'elles conviennent pour favoriser l'absorption de médicaments à base de protéine ou de peptide à travers le tissu des muqueuses vaginales.

6. Compositions pharmaceutiques suivant les revendications précédentes, caractérisées en ce qu'elles conviennent pour favoriser l'absorption de médicaments à base de protéine ou de peptide à travers le tissu des muqueuses nasales.

7. Compositions pharmaceutiques suivant les revendications précédentes, caractérisées en ce qu'elles conviennent pour favoriser l'absorption de médicaments à base de protéine ou de peptide à travers le tissu des muqueuses rectales.

8. Compositions pharmaceutiques suivant les revendications précédentes, caractérisées par la présence de copolymères polyoxyéthylène-polyoxypropylène comme agents thermodurcissables (capables d'augmenter la viscosité du produit au moyen de la transition de phase sol-gel induite par l'exposition à la température du site d'administration du corps.

9. Compositions pharmaceutiques suivant la revendication 8, dans lesquelles l'agent thermodurcissable est représenté par un copolymère connu sous la dénomination commerciale PLURONIC F127™, à une gamme de concentration finale comprise entre 10% et 30% en poids/poids, de préference entre 13% et 20% en poids/poids.

10. Compositions pharmaceutiques suivant les revendications précédentes, contenant un médicament à base de protéine ou de peptide choisi dans le groupe consistant en calcitonine, insuline, desmopressine, interleukine, interféron, GMCSF (facteur stimulant le développement de granulocytes monocytes) (granulocyte monocyte colony stimulating factor), cyclosporine, posatireline, protireline, timopentine, antigènes monoclonaux et polyclonaux, protéine antigénique bactérienne ou virale, parathormon, gonadoreline, facteurs de coagulation, facteurs de croissance épidermiques, facteur de croissance "analogue à l'insuline", endorphine et ses dérivés ou fragment, thioxoprolylcystéine, acide thioxoprolylthiazolidine carboxylique, nicotine, hirudine et/ou leurs dérivés.

11. Compositions pharmaceutiques suivant les revendications précédentes, dans lesquelles le médicament à base de protéine ou de peptide est représenté par une calcitonine.

12. Compositions pharmaceutiques suivant les revendications précédentes, dans lesquelles le médicament est représenté par un antigène ou un anticorps convenable pour être utilisé pour une immunothérapie.

13. Compositions pharmaceutiques suivant les revendications précédentes, contenant comme agents augmentant l'absorption ou stabilisants, un ou plusieurs des biopolymères parmi l'acide polygalacturonique, l'acide polyglycuronique, l'acide hyaluronique, l'hyaluronamide ou leurs sels ou dérivés.

14. Compositions pharmaceutiques suivant les revendications précédentes, susceptibles d'être administrées sous forme de mousse dermique ou vaginale.
